(19) Europäisches Patentamt
European Patent Office
Office européen des brevets

(11) Publication number: **0 138 358**
**A1**

(12) **EUROPEAN PATENT APPLICATION**

(21) Application number: **84305997.3**

(22) Date of filing: **31.08.84**

(51) Int. Cl.⁴: **C 07 D 405/04**, A 61 K 31/415, A 61 K 31/335

(30) Priority: **09.09.83 FI 833224**

(43) Date of publication of application: **24.04.85 Bulletin 85/17**

(84) Designated Contracting States: **AT BE CH DE FR GB IT LI LU NL SE**

(71) Applicant: **Farmos-Yhtymä Oy, P.O. Box 425, SF-20101 Turku 10 (FI)**

(72) Inventor: **Karjalainen, Arto Johannes, Myllyojantie 13 H 37, SF-90650 Oulo 65 (FI)**
Inventor: **Virtanen, Raimo Einari, Knaapintie 2-4 as.5, SF-21290 Rusko (FI)**

(74) Representative: **Collier, Jeremy Austin Grey et al, J.A.Kemp & Co. 14, South Square Gray's Inn, London WC1R 5EU (GB)**

(54) **The preparation and use of 2-2-(1,4-benzodioxanyl)l-2-imidazoline in therapy.**

(57) 2- [2- (1,4-Benzodioxanyl)] -2-imidazoline and its salts is useful in therapy as an antidote to $\alpha_2$-antagonists useful as veterinary sedatives or analgesics. It may advantageously be made by reacting 1,4-benzodioxane-2-carboxylic acid with ethylene diamine in a solvent which is insoluble in water and has a high boiling point. The water resulting from the reaction is continuously removed during the process.

EP 0 138 358 A1

# THE PREPARATION AND USE OF 2-[2-(1,4-BENZODIOXANYL)]-2-IMIDAZOLINE IN THERAPY

This invention relates to the preparation of 2-[2-(1,4-benzodioxanyl)]-2-imidazoline, and to its use.

Detomidine, which has the formula

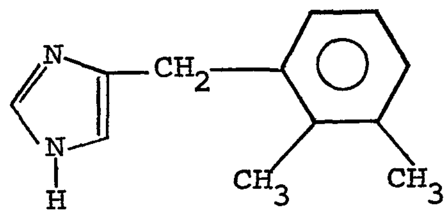

is a known compound with sedative and analgesic effect in animals. The potency and the duration of the effect can be regulated by the dosage. The compound is very potent; dosages as small as 15-20 µg/kg given by injection provide a clinical response. Detomidine is used in veterinary medicine especially in the handling of horses and cattle (pharmacological restraint). The animal is sedated before investigation, treatment and difficult medical operations. Even small surgical operations cannot be carried out without the use of a sedative agent.

The analgesic effect is dose dependent. Table 1 shows the effects produced.

Table 1: The effect of detomidine in horses and cattle. Dosage i.m. or i.v.

| Dose µg/kg ml/100 g | Effect | Delay before the effect begins (min) | Duration of the effect (hours) | Other effects |
|---|---|---|---|---|
| 20-40 0.2-0.4 | easy to handle; short effect | 3-5 | 1/2-1 | slight staggering |
| 40-80 0.4-0.8 | strong effect | 2-5 | 1/2-2 | staggering, sweating pilo-erection, muscular tremor |
| 80-150 0.8-1.5 | can be used if necessary | 1-5 | 2-6 | Strong staggering, pilo-erection, sweating, diuresis |

The effect of detomidine has been described in the literature, e.g. O. Vainio: "Detomidine hydrochloride - a novel imidazole-type sedative analgesic." Pharmacologie et Toxicologie Veterinaires, INRA Publ. Paris, 1982, Les Colloques de l'I.N.R.A., 8.

When the treatment using detomidine is finished, it is for practical reasons desirable to reverse its effect by a specific antagonist or antidote. The animal could then immediately be transported away from the surgery, and expensive awakening rooms are not

required. The ability of the animal to control its movements and co-ordination after awakening is improved. When animals are treated in cold surroundings this is absolutely necessary, because otherwise the animal would remain lying still for too long a time. Using an awakening agent, the feeding of cattle could start more rapidly than otherwise (interruption in feeding causes disturbance in production).

The use of an awakening agent in connection with the use of detomidine saves time for the veterinarian as well as for the owner of the animal. The antidote enables the use of higher doses of detomidine, which induce a stronger analgesic effect. Safety of the treatment of big animals is thus increased. Without any awakening agent, detomidine could not be used in some cases, as it is often not possible to wait until the animal has recovered from the influence of detomidine.

Detomidine is an $\alpha_2$-antagonist. Other $\alpha_2$-antagonists useful as veterinary sedatives or analgesics are xylazine, disclosed in U.S. Patent No. 3235550 and 4-(2,6-dimethylbenzyl)imidazole, a detomidine analogue disclosed in U.S. Patent No. 4443466.

The present invention thus provides 2-[2-(1,4-benzodioxanyl)]-2-imidazoline for use in therapy, especially veterinary therapy, as an antidote for $\alpha_2$-antagonists useful as veterinary sedatives and analgesics, e.g. detomidine, xylazine and 4-(2,6-dimethylbenzyl)imidazole.

For this purpose it may be presented as a pharmaceutical composition, suitable for administration by, more particularly, injection, especially intravenous injection, comprising 2-[2-[1,4-benzodioxanyl)]-2 imidazoline or a non-toxic salt thereof, e.g. the hydrochloride and a non-toxic pharmaceutically acceptable carrier.

It has now surprisingly been found that the compound 2-[2-(1,4-benzodioxanyl)]-imidazoline, which has the formula

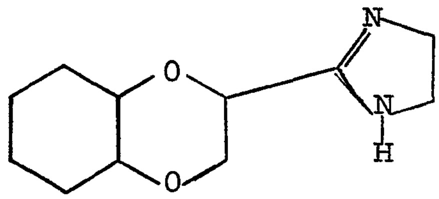

(I)

is a very good antidote for $\alpha_2$ antagonists useful as veterinary sedatives and analgesics, e.g. detomidine, xylazine and 4-(2,6-diemthylbenzyl)imidazole.

Trials were carried out as follows:

(A) A dose of 0.04-0.06 mg/kg of detomidine was injected i.m. into the test animals. After exactly 30 min. compound (I) was given intravenously. The awakening of the animal or its recovery from the sedation was then observed. Calves (8) and Cows (7) recovered from detomidine-induced sedation in 2 to 5 minutes from the injection of compound (I). Horses (4) awoke in 5 to 10 min. The awakening effect of compound (I) was found stable for both species. No side-effects were observed. For the calves and cows, the dose of compound (I) was the same as the dose of detomidine. For the horses, the dose of compound (I) was five times greater than that of detomidine.

(B) A dose of 2 mg/kg of xylazine was injected i.m. into 10 dogs. This injection induced sedation which lasted for 0.5 - 1 hr. During this time the dogs were able to walk only with difficulty.

In other experiments compound (I) was given at a dose of 0.1 - 0.3 mg/kg i.v. 20 min. after the injection of 2 mg/kg of xylazine. The awakening of the dogs and their recovery from the sedation was observed. The dogs recovered from the xylazine-induced sedation in 2 - 3 minutes after the administration of compound (I).

At this time the dogs were able to walk easily and proved to be fully normal in other respects. The awakening effect of compound (I) was found to be stable. No side-effects were observed.

(C) The following tests concerning the sedative effect of 4-(2,6-dimethylbenzyl)imidazole and the inhibition of this effect with compound (I) were carried out in rats.

A dose of 0.5 mg/kg of 4-(2,6-dimethylbenzyl) imidazole was injected into 10 rats. This dose induced loss of the righting reflex for 1 h.

In other tests, compound (I) was given i.v. or i.p. to rats at a dose of 1-3 mg/kg, 15 minutes after the administration of 4-(2,6-dimethylbenzyl)imidazole. The recovery of the animals from the sedation was observed by measuring their spontaneous locomotor activity with an activity meter. Given at a dose of 1-3 mg/kg compound (I) was able to fully counteract the sedative effect of 4-(2,6-dimethylbenzyl)imidazole in 1-5 min. after the administration of compound (I).

The compound (I) has been described in US-patent No. 2979511 and British patent specification 2068376A. The US patent 2979511 discloses a method in which the starting materials, 1,4-benzodioxane-2 carboxylic acid and ethylene diamine, are refluxed and water is removed intermittently by distillation. The reaction is the following:

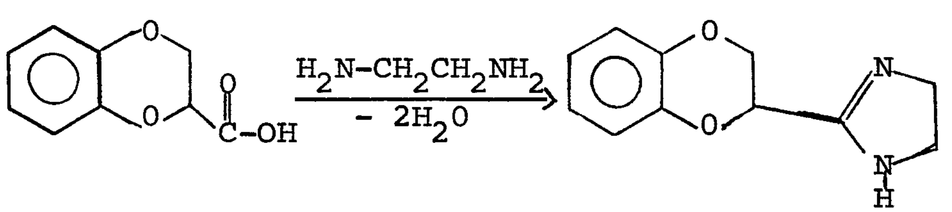


(I)

The compound was distilled as base, b.p. 155-165$^{o}$C/0.1 mm Hg, and converted into the corresponding hydrochloride, which had a melting point of 241-243$^{o}$C after recrystallisation from isopropanol.

British specifiction No. 2068376A discloses that the method described in the US-patent gives compound (I) only as a by-product and that the main product of the reaction is a compound which has the formula:

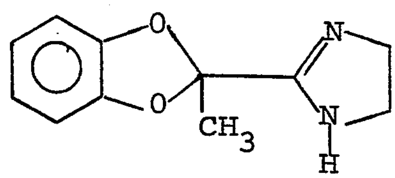

Repetition of the method described in US-patent 2979511 and thin layer analysis of the product shows that compound (I) is obtained only in extremely small amounts.

We have now found that the compound (I) can be prepared from the same starting materials as those used in US-patent No. 2979511, but in much better yield if the reaction is carried out in a solvent which is insoluble in water and has a high boiling point, preferably xylene, and the water resulting from the reaction is removed continuously during the process.

The reagents are preferably cautiously added dropwise to the boiling solvent. The process may be further improved by using a trialkylamine, e.g. triethylamine, as an additive which promotes the dissolution of 1,4-benzodioxane-2-carboxylic acid in xylene. A high temperature is essential for the process.

The following Example illustrates the invention.

EXAMPLE

3.5 g of 1,4-benzodioxane-2-carboxylic acid and 3.8 g of triethylamine are added to 30 ml of xylene. The mixture is stirred until the solid reagent has dissolved. 2.3 g of ethylene diamine are dissolved in another 30 ml of xylene. These solutions are added

simultaneously dropwise to 30 ml of xylene in a water separator. The mixture is refluxed for 10 hours. The reaction mixture is cooled and the xylene layer poured into cold diluted hydrochloric acid. The water layer is acidfied. The xylene is removed and the pH of the water layer is adjusted to 9-10. The removed oil is extracted in toluene. The combined toluene extracts are washed with water, dried and evaporated. 2.0 g of the product are obtained and it is pure compound (I) according to thin layer analysis.

The product is converted into the hydrochloride by HCl-ether in a mixture of isopropanol and ether. M.p. of the hydrochloride is 193-195 °C. The H-NMR-spectrum is identical with that of the product obtained by the process described in the British patent application 2068376A. The accompanying Fig 1 and Fig 2 show the H-NMR-spectra. Fig 1 shows the spectrum for the compound made according to the present invention and Fig 2 shows that of the compound made according to British patent application 2068376A. The solvent was $D_2O$ and the reference was the sodium salt of 3-(trimethylsilyl)-propane sulfonic acid.

CLAIMS

1. A process for the preparation of 2-[2-(1,4-benzodioxanyl)]-2-imidazoline which comprises reacting 1,4-benzodioxane-2-carboxylic acid with ethylene diamine in a solvent which is insoluble in water and has a high boiling point and removing the water resulting from the reaction continuously during the process.

2. A process according to claim 1, in which the reagents are slowly added to the boiling solvent.

3. A process according to claim 1 or 2 in which the solvent is xylene.

4. A process according to any one of claims 1 to 3 in which an additive is included in the reaction mixture to promote the dissolution of 1,4-benzodioxane-2-carboxylic acid in xylene.

5. A process according to claim 4, in which the additive is triethylamine.

6. 2-[2-(1,4-Benzodioxanyl)]-2-imidazoline and its non-toxic salts for use in therapy as a antidote to $\alpha_2$-antagonists useful as veterinary sedativies or analgesics.

7. 2-[2-(1,4-benzodioxanyl)]-2-imidazoline and its non-toxic salts for use in therapy as a detomidine antidote.

8. 2-[2-(1,4-benzodioxanyl)]-2-imidazoline and its non-toxic salts for use in therapy as a xylazine antidote.

9. A pharmaceutical composition comprising 2-[2-(1,4-benzodioxanyl)]-2-imidazoline or a non-toxic salt thereof, and a non-toxic, pharmaceutically acceptable carrier therefor.

10. A composition according to claim 9 in which the salt is the hydrochloride.

11. A composition according to claim 9 or 10 in unit dosage form.

| NO. | CURSOR | FREQ | PPM | INTEGRAL | INTENSITY |
|---|---|---|---|---|---|
| 1 | 2285 | 569.490 | 7.107 | .191 | 1.101 |
| 2 | 2312 | 564.874 | 7.049 | 1.298 | 8.106 |
| 3 | 2335 | 560.942 | 7.000 | .279 | 1.591 |
| 4 | 3011 | 445.369 | 5.558 | .099 | .601 |
| 5 | 3033 | 441.607 | 5.511 | .200 | 1.187 |
| 6 | 3054 | 438.017 | 5.466 | .118 | .736 |
| 7 | 3418 | 375.785 | 4.690 | 2.125 | 24.000 |
| 8 | 3447 | 370.827 | 4.628 | .042 | .521 |
| 9 | 3499 | 361.937 | 4.517 | .549 | 4.814 |
| 10 | 3520 | 358.346 | 4.472 | .443 | 3.603 |
| 11 | 3742 | 320.392 | 3.998 | 1.955 | 12.829 |
| 12 | 4571 | 178.660 | 2.230 | .063 | .732 |
| 13 | 5616 | 0.000 | 0.000 | .235 | 2.781 |

Fig.1.

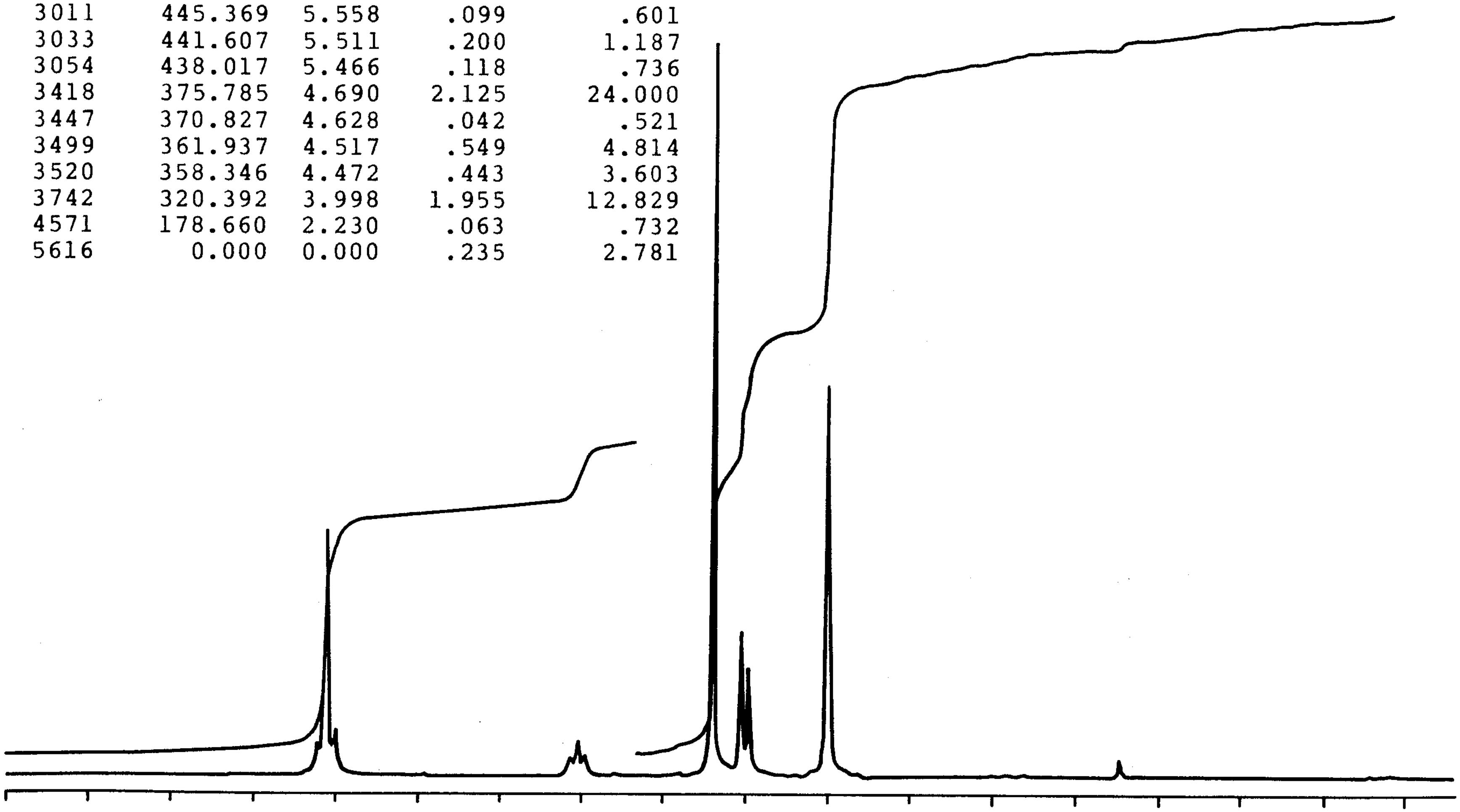

Fig. 2.

| NO. | CURSOR | FREQ | PPM | INTEGRAL | INTENSITY |
|---|---|---|---|---|---|
| 1 | 2277 | 569.149 | 7.103 | .273 | .780 |
| 2 | 2282 | 568.294 | 7.092 | .066 | .623 |
| 3 | 2304 | 564.532 | 7.045 | 1.732 | 5.429 |
| 4 | 2326 | 560.771 | 6.998 | .210 | .942 |
| 5 | 2334 | 559.403 | 6.981 | .134 | .513 |
| 6 | 3001 | 445.369 | 5.558 | .150 | .417 |
| 7 | 3021 | 441.949 | 5.515 | .276 | .767 |
| 8 | 3044 | 438.017 | 5.466 | .183 | .491 |
| 9 | 3396 | 377.837 | 4.715 | .259 | .659 |
| 10 | 3418 | 374.075 | 4.668 | 3.951 | 24.000 |
| 11 | 3445 | 369.459 | 4.611 | .173 | .515 |
| 12 | 3490 | 361.766 | 4.515 | .723 | 3.420 |
| 13 | 3511 | 358.175 | 4.470 | .682 | 2.380 |
| 14 | 3733 | 320.221 | 3.996 | 1.342 | 8.239 |
| 15 | 3736 | 319.708 | 3.990 | 1.331 | 7.897 |
| 16 | 4016 | 271.837 | 3.392 | .111 | .488 |
| 17 | 5040 | 96.767 | 1.208 | .086 | .461 |
| 18 | 5076 | 90.612 | 1.131 | .092 | .447 |
| 19 | 5606 | .000 | .000 | .544 | 2.706 |

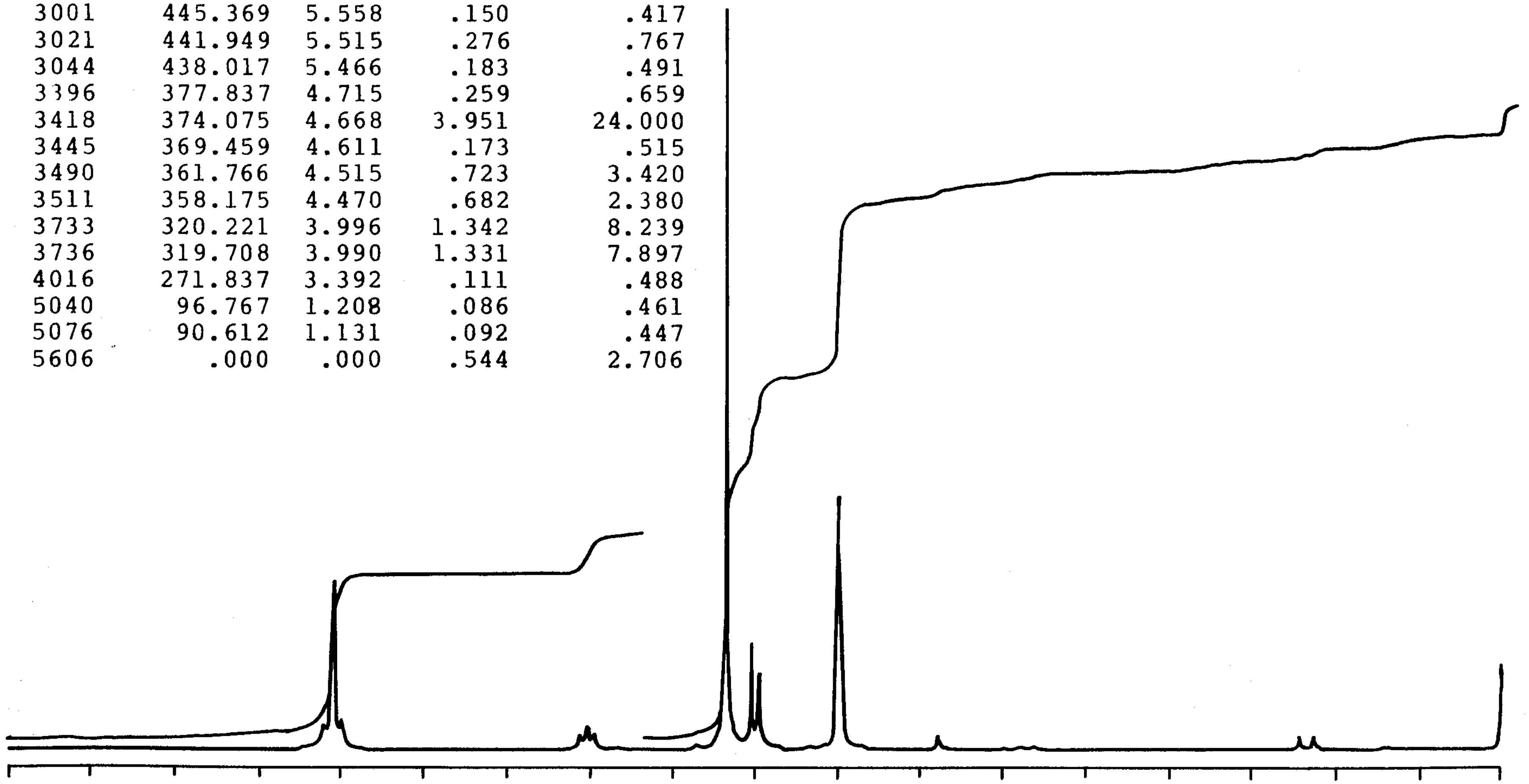

European Patent Office

# EUROPEAN SEARCH REPORT

0138358

EP 84 30 5997

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 047 531 (SYNTEX)<br>--- | | C 07 D 405/04<br>A 61 K 31/415<br>A 61 K 31/335 |
| D,A | US-A-2 979 511 (KRAPCHO-LOTT)<br>----- | | |
| | | | **TECHNICAL FIELDS SEARCHED (Int. Cl.4)** |
| | | | C 07 D 405/00<br>A 61 K 31/00 |

The present search report has been drawn up for all claims

| Place of search | Date of completion of the search | Examiner |
|---|---|---|
| THE HAGUE | 03-12-1984 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document